# EUROPÄISCHE PATENTANMELDUNG

(11) **EP 1 736 779 A2**
(43) Veröffentlichungstag der Anmeldung: **27.12.2006**
(21) Anmeldenummer: 06012291.8
(22) Anmeldetag: 14.06.2006
(51) Int. Cl.: G01N 33/543, G01N 33/532, C12Q 1/68, B01J 19/00, C40B 50/18, C40B 70/00, C40B 80/00, C40B 99/00

(54) **Verfahren zur Qualitätskontrolle funktionalisierter Oberflächen**

(30) Priorität: 15.06.2005 DE 102005027667
(71) Anmelder: febit biotech GmbH, 69120 Heidelberg (DE)
(72) Erfinder: Scheffler, Matthias, Dipl.-Chem.Dr., 69493 Hirschberg (DE)
(74) Vertreter: Weiss, Wolfgang

(57) **Zusammenfassung**

Die Erfindung betrifft ein Verfahren, mit dem sich einerseits eine Reaktion und andererseits die Verteilung funktioneller Gruppen auf einer Oberfläche überprüfen lässt. Dieses Verfahren kommt zum Einsatz, wenn es darauf ankommt, eine möglichst homogene Verteilung der Oberflächen-Funktionalisierung zu dokumentieren oder zu überprüfen.

## Beschreibung

Die Erfindung betrifft ein Verfahren, mit dem sich einerseits eine Reaktion und andererseits die Verteilung funktioneller Gruppen auf einer Oberfläche überprüfen lässt. Dieses Verfahren kommt zum Einsatz, wenn es darauf ankommt, eine möglichst homogene Verteilung der Oberflächen-Funktionalisierung zu dokumentieren oder zu überprüfen. Die Funktionalisierung erfolgt mit einem für das Oberflächenmaterial geeigneten Beschichtungsverfahren und ―molekülen. Insbesondere dient das erfindungsgemäße Verfahren der Qualitätskontrolle bei der Herstellung von Reaktionsträgern für so genannte Biochips, Gen-Chips und Microarrays.

Die Herstellung von Reaktionsträgern bzw. Arrays umfasst zunächst eine Funktionalisierung des Trägers, wobei funktionelle Gruppen im Allgemeinen in einem Beschichtungsprozess auf die Oberfläche aufgetragen werden. Die funktionalisierten Oberflächen stehen dann in einem nächsten Bearbeitungsschritt für die weitere Beschichtung mit entsprechenden biologisch oder chemisch funktionellen Materialien resp. Molekülen zur Verfügung. Beispiele für diese biologisch oder chemisch funktionellen Materialien resp. Moleküle sind Nukleinsäuren oder Nukleinsäureanaloga, wie DNA, RNA, PNA, LNA, Oligonukleotide (unabhängig von der Syntheserichtung), Saccharide bzw. Kohlenhydrate, Peptide, Proteine sowie weitere Mischformen, aber auch Derivate aus der kombinatorischen Chemie oder Linkermoleküle bzw. Bausteine zur Synthese solcher Moleküle.

Ein Problem dabei ist jedoch, dass die Qualität der funktionalisierten Oberfläche nicht ausreichend genau bestimmt werden kann.

Ein Gegenstand der vorliegenden Erfindung ist ein Verfahren zur Überprüfung funktionalisierter Gruppen auf einer Oberfläche,
(a) Bereitstellen eines Trägers, umfassend eine Oberfläche,
(b) Beschichten zumindest eines Teils der Oberfläche des Trägers mit ersten funktionellen Beschichtungsmolekülen, wobei man erste Beschichtungsmoleküle verwendet, die eine reversibel gebundene Markierung tragen,
(c) Nachweis der Existenz und/oder Dichte oder/und Verteilung der Markierungen auf der Oberfläche,
(d) Abspalten der Markierungen und
(e) gegebenenfalls Beschichten des Trägers mit weiteren funktionellen Beschichtungsmolekülen, die mit funktionellen Gruppen der ersten Beschichtungsmoleküle reagieren, wobei die funktionellen Gruppen vorzugsweise durch Abspaltung der Markierungsgruppen freigesetzt worden sind.

Ein weiterer Gegenstand der Erfindung ist ein Reagenz insbesondere zur Durchführung des oben genannten Verfahrens, enthaltend zumindest ein erstes funktionelles Beschichtungsmolekül mit einer reversibel gebundenen Markierung und zumindest ein weiteres funktionelles Beschichtungsmolekül, das mit funktionellen Gruppen des ersten Beschichtungsmoleküls reagieren kann, wobei die funktionellen Gruppen vorzugsweise durch Abspaltung der Markierungsgruppen freigesetzt worden sind.

Die Funktionalisierung der Träger kann mit üblichen Reagenzien erfolgen, z.B. mit Silanisierungsreagenzien für Glasträger. Erfindungsgemäß tragen die zur Funktionalisierung verwendeten Moleküle eine Markierung oder mehrere Markierungen, die sich durch geeignete Verfahren detektieren lassen. Zur Analyse der Markierung können dem entsprechend Absorption, Emission, Radioaktivität, Plasmonenresonanz, Lichtbeugung, Lichtstreuung, Ellipsomerie, Elektronenbeugung oder elektrische Signale etc. herangezogen werden. Bevorzugt sind optisch messbare Marker wie Fluorophore. Bevorzugt sind Silane, die mindestens eine, z.B. eine oder zwei funktionelle Gruppen zur Reaktion mit biologischen Substanzen und mindestens eine Markierung tragen. Besonders bevorzugt sind Funktionalisierungsreagenzien, die eine Silangruppe zur Bindung an Glasoberflächen und einen Baustein zur Nukleinsäuresynthese, z.B. eine Phosphoramiditgruppe, enthalten.

Die Erfindung wird insbesondere in Verfahren eingesetzt, die einen schrittweisen Aufbau von Rezeptoren, vorzugsweise von mehreren unterschiedlichen Rezeptoren auf einem einzigen Träger, aus mehreren Synthesebausteinen umfassen. Der Träger kann ein beliebiger Träger, z.B. ein Träger mit planarer oder strukturierter Oberfläche, sein.

Vorzugsweise ist der Träger ein mikrofluidischer Träger, insbesondere ein Träger mit geschlossenen Kanälen, auf deren Oberfläche die Rezeptorsythese erfolgt. Bevorzugte Träger sind z.B. in WO 00/13018 und WO 00/13017 beschrieben.

Die Markierung muss sich durch geeignete Reaktionsführung selektiv und im besonderen ortsspezifisch von den Beschichtungsmolekülen entfernen lassen, z.B. durch chemische und/oder photochemische Methoden. Die ortsspezifische Freisetzung des Reaktionszentrums erlaubt den unabhängigen Aufbau von funktionellen Molekülen, z.B. Sonden (unabhängig von der Sondensequenz und/oder Sondenart, z.B. Oligonukleotide, Peptide, etc.). Besonders bevorzugt sind Moleküle, die in der Anmeldung PCT/EP03/14826 "Verfahren zum validierten Aufbau von Arrays" offenbart sind, insbesondere die Typen I, II, VI und VII. Weiterhin bevorzugt sind zweistufige Schutzgruppen, insbesondere modifizierte Tritylgruppen, wie sie in WO 03/004510 beschrieben sind. Auf den Inhalt dieser Dokumente wird ausdrücklich Bezug genommen.

Die Signale, die von den Markierungsgruppen stammen, werden nach einer Datenanalyse zur Beurteilung der Homogenität der Oberflächenbelegung herangezogen. Anschließend werden zum Aufbau funktioneller Moleküle die Markierungsgruppen durch chemische Reagenzien und/oder durch Bestrahlung abgespalten.

Durch die Erfindung wurden funktionalisierte Träger mit messbaren Oberflächencharakteristika bereitgestellt, die als Ausgangsmaterialien zum Aufbau von Reaktionsträgern bzw. Arrays dienen können.

Spezielle Ausführungsbeispiele, die auf Silanen und Fluoreszenzfarbstoffen basieren:

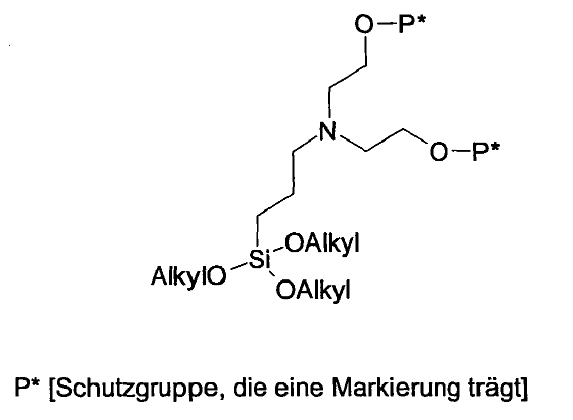

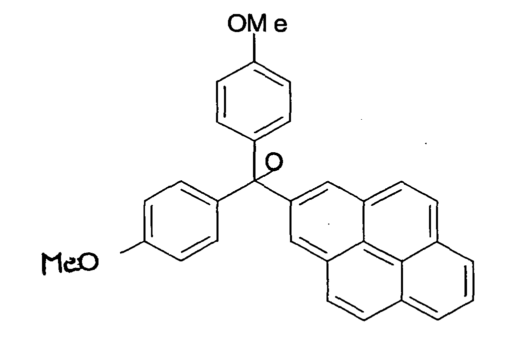

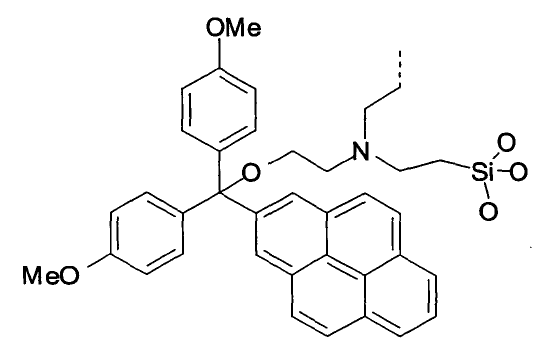

Neben Fluoreszenzfarbstoffen, wie z.B. Pyrenen, Coumarinen, Rhodaminen, Cyaninen, kommen auch Nanopartikel und andere Fluoreszenzmarkierungen in Betracht.

## Patentansprüche

1. Verfahren zur Überprüfung funktionalisierter Gruppen auf einer Oberfläche,
(a) Bereitstellen eines Trägers, umfassend eine Oberfläche,
(b) Beschichten zumindest eines Teils der Oberfläche des Trägers mit ersten funktionellen Beschichtungsmolekülen, wobei man erste Beschichtungsmoleküle verwendet, die eine reversibel gebundene Markierung tragen,
(c) Nachweis der Existenz und/oder Dichte oder/und Verteilung der Markierungen auf der Oberfläche,
(d) Abspalten der Markierungen und
(e) gegebenenfalls Beschichten des Trägers mit weiteren funktionellen Beschichtungsmolekülen, die mit funktionellen Gruppen der ersten Beschichtungsmoleküle reagieren, wobei die funktionellen Gruppen vorzugsweise durch Abspaltung der Markierungsgruppen freigesetzt worden sind.

2. Verfahren nach Anspruch 1 zur Herstellung von Trägern für Biochips, Gen-Chips und Mikroarrays.

3. Verfahren nach Anspruch 1 oder 2,
**dadurch gekennzeichnet,**
**dass** die weiteren funktionellen Beschichtungsmoleküle ausgewählt werden aus:
(a) Nukleinsäuren oder Nukleinsäureanaloga oder Bausteine zu deren Synthese,
(b) Peptiden oder Proteinen oder Bausteine zu deren Synthese,
(c) Kohlenhydraten oder Bausteinen zu deren Synthese,
(d) Verbindungen aus der kombinatorischen Chemie oder Bausteinen zu deren Synthese und
(e) Linkermolekülen oder Bausteinen zu deren Synthese.

4. Reagenz zur Durchführung des Verfahrens nach einem der Ansprüche 1 bis 3, enthaltend zumindest ein erstes funktionelles Beschichtungsmolekül mit einer reversibel gebundenen Markierung und zumindest ein weiteres funktionelles Beschichtungsmolekül, das mit funktionellen Gruppen des ersten Beschichtungsmoleküls reagieren kann, wobei die funktionellen Gruppen vorzugsweise durch Abspaltung der Markierungsgruppen freigesetzt worden sind.
